**Europäisches Patentamt**

**European Patent Office**

**Office européen des brevets**

(19)

(11) Veröffentlichungsnummer: **0 429 006 A1**

(12) **EUROPÄISCHE PATENTANMELDUNG**

(21) Anmeldenummer: **90121875.0**

(22) Anmeldetag: **15.11.90**

(51) Int. Cl.⁵: **C12N 1/14, A01G 1/04, B27K 9/00**

(30) Priorität: **21.11.89 DE 3938659**

(43) Veröffentlichungstag der Anmeldung:
**29.05.91 Patentblatt 91/22**

(84) Benannte Vertragsstaaten:
**AT BE CH DE DK ES FR GB GR IT LI LU NL SE**

(71) Anmelder: **REKO GMBH, ENTSORGUNG UND RECYCLING**
**Gutenbergstrasse 53**
**W-7123 Sachsenheim 1(DE)**

(72) Erfinder: **Grothey, Volker, Dipl.-Landwirt**
**Sonnenbreite 1**
**W-3400 Göttingen(DE)**
Erfinder: **Majcherczyk, Andrzej**
**Thiestrasse 3a**
**W-3400 Göttingen(DE)**
Erfinder: **Hüttermann, Aloys, Prof. Dr.**
**Henry-Dunant-Strasse 20**
**W-3400 Göttingen(DE)**

(74) Vertreter: **Harders, Gerhard, Dr.**
**Stettiner Strasse 2**
**W-6367 Karben 6(DE)**

(54) **Verfahren zum Aufbereiten von Stroh als Substrat für die Anzucht von Pilzkulturen.**

(57) Ein Verfahren zum Aufbereiten von Stroh als Substrat für Pilzkulturen wird durchgeführt, indem
a) das Stroh in einem Reaktionsgefäß bis zur Sättigung mit Wasser befeuchtet wird"
b) das befeuchtete Stroh mit Wasser von oben besprüht wird, wobei die unten abtropfende Wassermenge im Kreisverfahren von oben wieder aufgesprüht wird;
c) nach einer Sprühzeit von mindestens 3 bis 100 Stunden das behandelte feuchte Stroh entnommen und gegen frisches Stroh ausgetauscht wird, wobei die mit dem Stroh entnommene Wassermenge im System ergänzt wird.
Das so aufbereitete Stroh kann für die Anzucht von Speiespilzen oder für die Anzucht von Mycelien für die Reinigung von Gasen oder Entsorgung kontaminierter Böden verwendet werden.

FIG. 1

Die vorliegende Erfindung betrifft ein Verfahren zum Aufbereiten von Stroh als Kulturmedium für Pilzkulturen.

Als Substrat für die Anzucht von bestimmten Pilzen, wie z.B. den Speisepilz Pleurotus, kann Stroh verwendet werden. Das Stroh wird für die Verwendung als Substrat zerkleinert, angefeuchtet und wärmebehandelt, was auch als Fermentation bezeichnet wird (siehe z. B. "Practical Problems in the Pretreatment of Straw-Based Lignocellulosic Substrate for Pleurotus Production", J. Eder, in: Treatment of Lignocellulosics with White Rot Fungi, Elsevier Applied Science, London und New York, 1988, S. 14-20).

In neuerer Zeit ist Stroh als Substrat für Speisepilze auch "semianaerob" fermentiert worden (U. Schies und J. Lelley, in: "Mushroom Science", XII, 1989,IIs. 199-205 ). Als semianaerob bezeichnet man eine Fermentation unter Wasser, also bei begrenzter Sauerstoffzufuhr. Dabei wurde festgestellt, daß das Fermentationswasser offenbar ein antimykotisch wirksames Prinzip enthält, das das Wachstum der Kulturpilze hemmt.

Es wurde gefunden, daß bei der Aufbereitung von Stroh als Substrat für lignolytische Pilze durch Fermentation Phenole freigesetzt werden (Europ. J. Appl. Microbiol. Biotechnol., 1981, 12, S. 183-188). Phenole sind als starke Zellgifte bekannt.

Es war daher zu erwarten, daß bei der Fermantation von Stroh für die Verwendung als Substrat für die Anzucht von Kulturpilzen das Fermentationswasser sich bei der Wiederverwendung mit Stoffen anreichern würde,, die dem Substrat ungünstige Eigenschaften für das Wachstum der Kulturpilze verleihen würden.

Aufgabe der vorliegenden Erfindung ist es, ein Fermantationsverfahren für Stroh als Substrat für die Anzucht von Kulturpilzen zu schaffen, das nur einen geringen Wasserbedarf aufweist und abwasserfrei ist.

Gelöst wird diese Aufgabe durch ein Verfahren mit den im Kennzeichen des Hauptanspruchs angegebenen Maßnahmen. Die Unteransprüche betreffen bevorzugte Ausgestaltungen der Erfindung.

Bei der erfindungsgemäßen Wasserbehandlung wird das Stroh nur eine verhältnismäßig kurze Zeit bis zu etwa 100 Stunden mit Wasser in Berührung gebracht. Dabei wird offenbar im Gegensatz zu der vorher beschriebenen semianaeroben Fermentation, bei der die Behandlungszeiten 7 Tage und mehr betragen, noch keine stärkere Hydrolyse des Strohs erreicht, sondern in erster Linie lediglich eine Quellung, was aber überraschenderweise für die Verwendung des so behandelten Strohs als Substrat für die Anzucht von Kulturpilzen völlig ausreichend ist. Überraschenderweise ist beim erfindungsgemäßen Verfahren keine Anreicherung von antimykotisch wirksamen Stoffen im behandlungswasser festzustellen, sondern es stellt sich ein stationäres Gleichgewicht des Extraktstoffe ein, das dem Wachstum von Kulturpilzen nicht abträglich ist. Infolgedessen kann das zur Behandlung verwendete Wasser nach Beendigung der Behandlung im System verbleiben und es muß nur die mit dem Stroh entnommene Wassermenge ergänzt werden. Führt man auch das aus dem entnommenen Stroh abtropfende Wasser in das System zurück, so ist das Verfahren völlig abwasserfrei. Auch zur Reinigung verwendetes Spülwasser und dergleichen kann in das System zurückgeführt werden.

Die Behandlungszeiten sind wesentlich kürzer als bei der semianaeroben Fermentation. Schon nach 3 Stunden Sprühzeit kann ein Strohprodukt erhalten werden, das als Substrat geeignet ist. Sprühzeiten bis zu etwa 100 Stunden sind nur in ungünstigen Ausnahmefällen erforderlich, bevorzugt werden Sprühzeiten von etwa 6 bis 75 Stunden verwendet, besonders bevorzugt von etwa 12 bis 48 Stunden.

Es hat sich als günstig erwiesen, wenn dem verwendeten Spülwasser detergentien zugesetzt werden. Die Konzentration der Detergentien im Spülwasser soll etwa 10 bis 10 000 ppm betragen, bevorzugt etwa 50 bis 5000 ppm und besonders bevorzugt etwa 100 bis 500 p3m.

Es hat sich als besonders zweckmäßig erwiesen, wenn das Sprühwasser zusätzlich mit Fungiziden versetzt wird, um Kontaminationen während der Behandlung zu vermeiden. Die Konzentration der Fungizide kann etwa 1 bis 500 ppm betragen. Es können auch Suspensionen in höheren Konzentrionen eingesetzt werden. Ein bevorzugter Bereich bei Verwendung von Suspensionen liegt bei etwa 20 bis 200 ppm. Als Fungizig hat sich insbesondere Benomyl bzw. (Methyl)-1-(methylcarbamoyl)-2-benzimidazolcarbamat als geeignet erwiesen.

Durch die Möglichkeit, das Spülwasser in einem Kreisprozeß wiederzuverwenden, läßt sich eine Abwasserbelastung völlig vermeiden. Während bei der semianaeroben Fermentation eine etwa 15fache Abwassermenge, bezogen auf die Menge des fermentierten Strohs, anfällt, ist das erfindungsgemäße Verfahren völlig abwasserfrei.

Das erfindungsgemäße Verfahren läßt sich sowohl in Gegenwart von Sauerstoff als auch unter Sauerstoff-Ausschluß durchführen. Bei einer Begasung des Reaktionsgefäßes mit Sauerstoff oder Stickstoff werden keine signifikant abweichenden Ergebnisse erhalten.

Die Erfindung wird nachfolgend anhand der Zeichnungen in einem Ausführungsbeispiel beschrieben.

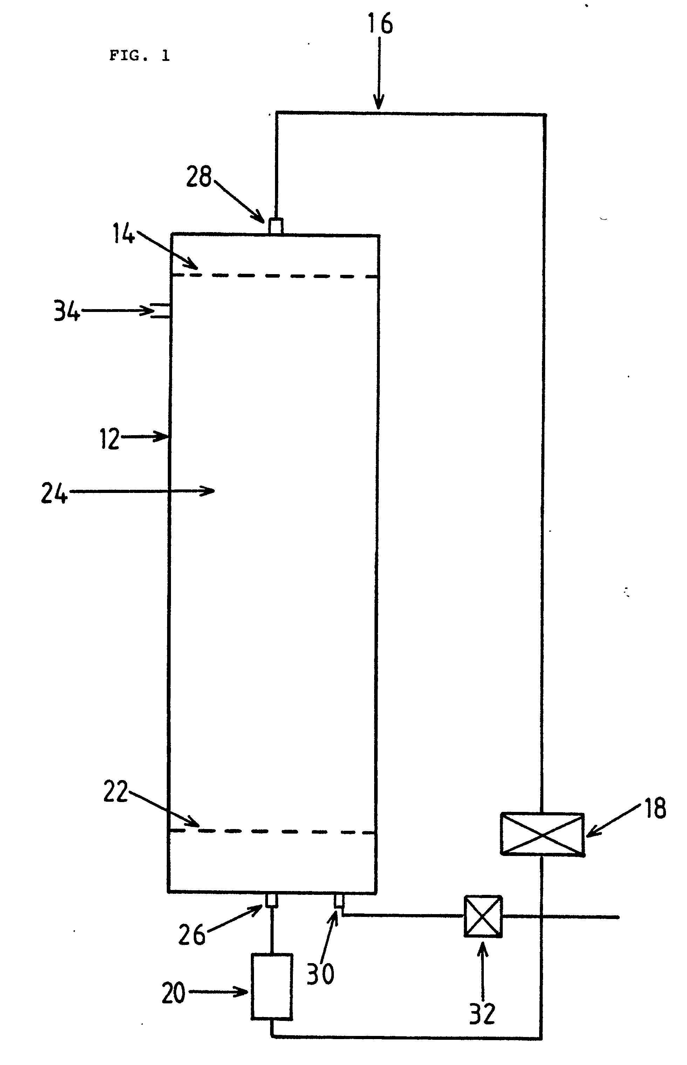

Fig. 1 zeigt eine schematische Zeichnung der Vorrichtung zum Behandeln von Stroh nach dem erfindungsgemäßen Verfahren.

Der innere Strohraum 24 eines Reaktionsgefäßes 12 ist mit einem unteren Sieb 22 versehen, auf das das zu behandelnde Stroh aufgeschichtet wird. Im oberen Teil des Reaktionsgefäßes 12 ist eine Sprühvorrichtung 14 angeordnet, über die Sprühwasser über einen Flüssigkeitseinlauf 28 eingesprüht wird. Das Sprühwasser verläßt das Reaktionsgefäß 12 nach dem Durchlauf durch das zu behandelnde Stroh durch einen unteren Flüssigkeitsablauf 26 und wird über eine Filtervorrichtung 20 zu einer Umwälzpumpe 18 geleitet, die das Sprühwasser wieder über die Verbindungsleitung 16 zum Flüssigkeitseinlauf 28 zurückführt.

Durch eine im unteren Teil des Reaktionsgefäßes 12 angebrachte Gaseintrittsöffnung 30 kann über einen Gasregler 32 Gas in das Reaktionsgefäß eingeleitet werden, das nach dem Durchströmen des Reaktionsgefäßes 12 aus der oberen Gasaustrittsöffnung 34 wieder austritt.

In der beschriebenen Vorrichtung wird das Verfahren wie folgt durchgeführt:

In daz zylindrische Reaktionsgefaß 12 mit einer Höhe von 1 m und einem Durchmesser von 30 cm wurde 1 kg gehäckseltes lufttrockenes Weizenstroh eingefüllt und von Hand mit leichtem Druck angedrückt, sodaß das Reaktionsgefäß bis nahe an die Gasaustrittsöffnung gefüllt war. Durch den Flüssigkeitseinlaß wurden 5 l Leitungswasser, die mit 70 ppm Benomyl und 500 ppm Detergenz Arquart 2 HT 75 versetzt waren, eingefüllt. Dann wurde der Flüssigkeitseinlaß verschlossen und die Umwälzpumpe eingeschaltet. Das unten abtropfende Spülwasser wurde in einer Menge von etwa 1 l/min abgepumpt und von oben wieder aufgesprüht. Die Sprühzeit betrug insgesamt etwa 24 Stunden.

Nach 24 Stunden Sprühzeit wurde die Pumpe abgeschaltet und das behandelte Stroh in dem Reaktionsgefäß 12 Stunden abtropfen gelassen. Dann wurde das behandelte Stroh, das auf 1 Gew.-Teil Stroh etwa 3 Gew.-Teile Wasser enthielt entnommen, das im Gefäß verbliebene Restwasser mit Leitungswasser auf eine Gesamtmenge von 5 l aufgefüllt und erneut 1 kg Stroh für einen zweiten Behandlungszyklus eingegeben. Der zweite Behandlungszyklus wurde ebenso wie noch 3 weitere folgende Zyklen, also insgesamt 5 Zyklen, wie der erste behandlungszyklus durchgeführt, wobei jeweils die ergänzend zugegebenen Wassermengen entsprechen dem Zyklus 1 mit Deterentien und Fungizid versetzt wurden.

Bereits nach dem 3. Behandlungszyklus stellte sich ein Gleichgewicht der extrahierten Substanzen, vor allem Phenole, im Sprühwasser ein.

1 kg des so behandelten Strohs wurden angeimpft mit einer 4 Wochen alten Kultur von Pleurotus spec. auf 60 g Stroh und in einen Plastiksack gefüllt. Der Sack wurde verschlossen und bei 250°C im Dunkeln stehen gelassen. Nach 4 Wochen war das gesamte Stroh deutlich mit Mycel durchwachsen.

Das so behandelte Stroh läßt sich beispielsweise als Kultursubstrat für die Anzucht von Speisepilzen oder für die Anzucht von Mycelien für die Reinigung von Gasen nach DE-A- 38 07 033 oder die Entsorgung kontaminierter Böden nach DE-A 37 311 816 verwenden.

**Ansprüche**

1. Verfahren zum Aufbereiten von Stroh als Substrat für Pilzkulturen,
**dadurch gekennzeichnet,** daß
   a) das Stroh in einem Reaktionsgefäß bis zur Sättigung mit Wasser befeuchtet wird;
   b) das befeuchtete Stroh mit Wasser von oben besprüht wird, wobei die unten abtropfende Wassermenge im Kreisverfahren von oben wieder aufgesprüht wird;
   c) nach einer Sprühzeit von mindestens 3 bis 100 Stunden das behandelte feuchte Stroh entnommen und gegen frisches Stroh ausgetauscht wird, wobei die mit dem Stroh entnommene Wassermenge im System ergänzt wird.

2. Verfahren nach Anspruch 1, dadurch gekennzeichnet, daß die Sprühzeit etwa 6 bis 75 Stunden beträgt.

3. Verfahren nach Anspruch 1, dadurch gekennzeichnet, daß die Sprühzeit etwa 12 bis 48 Stunden beträgt.

4. Verfahren nach Anspruch 1, dadurch gekennzeichnet, daß das Sprühwasser Detergentien in einer Menge von 10 bis 10 000 ppm enthält

5. Verfahren nach Anspruch 1 und 4, dadurch gekennzeichnet, daß der Detergentiengehalt 50 bis 5000 ppm beträgt.

6. Verfahren nach Anspruch 1 und 4, dadurch gekennzeichnet, daß der Detergentiengehalt 100 bis 500 ppm beträgt.

7. Verfahren nach Anspruch 1, dadurch gekennzeichnet, daß das Sprühwasser Fungizide in einer Menge von 1 bis 500 ppm enthält.

8. Verfahren nach Anspruch 1 und 7, dadurch gekennzeichnet, daß der Fungizidgehalt 20 bis 200 ppm beträgt.

9. Verwendung des nach den Ansprüchen 1 bis 8 aufbereiteten Strohs für die Anzucht von Speisepilzen oder für die Anzucht von Mycelien für die Reinigung von Gasen oder Entsorgung kontaminierter Böden.

FIG. 1

EP 0 429 006 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | FR-A-2 248 775 (LICENCIA TALALMANYOKAT ERTEKE-SITO VALLALAT) <br> * Seite 6, Zeile 34 - Seite 9, Zeile 16; Ansprüche * <br> - - - - - | 1-3,7-8 | C 12 N <br> 1/14 <br> A 01 G 1/04 <br> B 27 K 9/00 |

| | RECHERCHIERTE SACHGEBIETE (Int. Cl.5) |
|---|---|
| | C 12 N <br> A 01 G <br> B 27 K |

Der vorliegende Recherchenbericht wurde für alle Patentansprüche erstellt

| Recherchenort | Abschlußdatum der Recherche | Prüfer |
|---|---|---|
| Den Haag | 22 Februar 91 | BEVAN S.R. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument